Europäisches Patentamt

European Patent Office

Office européen des brevets

(11) Veröffentlichungsnummer: **0 185 868**
**B1**

(12) **EUROPÄISCHE PATENTSCHRIFT**

(45) Veröffentlichungstag der Patentschrift: **25.04.90**

(21) Anmeldenummer: **85112963.5**

(22) Anmeldetag: **12.10.85**

(51) Int. Cl.$^5$: **B 01 J 10/00, B 01 J 19/24 //**
**C07C17/02**

(54) Verfahren und Vorrichtung zur Durchführung von heterogenen, stofftransportlimitierenden Reaktionen.

(30) Priorität: **15.12.84 DE 3445904**

(43) Veröffentlichungstag der Anmeldung:
**02.07.86 Patentblatt 86/27**

(45) Bekanntmachung des Hinweises auf die
Patenterteilung:
**25.04.90 Patentblatt 90/17**

(84) Benannte Vertragsstaaten:
**AT BE CH DE FR GB IT LI LU NL SE**

(56) Entgegenhaltungen:
**EP-A-0 000 902**
**EP-A-0 142 016**
**FR-A-2 282 936**

(73) Patentinhaber: **HÜLS AKTIENGESELLSCHAFT**
**Paul-Baumann-Strasse 1**
**D-4370 Marl 1 (DE)**

(72) Erfinder: **Piotrowski, Bernhard, Dr.**
**Alte Lohmarer Strasse 84**
**D-5204 Lohmar (DE)**
Erfinder: **Hofmann, Gerhard**
**Alter Weg 7**
**D-5206 Neunkirchen-Seelscheid 1 (DE)**

Courier Press, Leamington Spa, England.

**Beschreibung**

Die Erfindung betrifft ein Verfahren und eine Vorrichtung zur Durchführung von heterogenen, stofftransportlimitierenden Reaktionen gemäß dem Oberbegriff des Patentanspruchs 1.

Die Direktchlorierung von Ethylen und Chlor zu 1.2-Dichlorethan (EDC) in einem flüssigen, katalysatorhaltigen Produktstrom aus EDC kann beispielhaft für den Reaktionstyp einer heterogenen, exothermen und stofftransportlimitierenden Reaktion angesehen werden (Ullmanns Enzyklopädie der technischen Chemie, 4. Aufl., Bd. 9 [1975], Seite 426—427).

Der Reaktionsumsatz, d.h. der Umsatz der Reaktanden zu dem gewünschten Produkt, und die Selektivität einer Reaktion können durch Maßnahmen zur Verbesserung des Stoff- und Wärmetransports verbessert werden. So werden z.B. auch bei den bekannten Vorrichtungen zur Durchführung der Direktchlorierung zu EDC Reaktionsbedingungen angestrebt, bei denen die Reaktanden und hierbei insbesondere Ethylen in möglichst einheitlicher, feinverteilter Gas/Flüssig-Dispersion vorliegen.

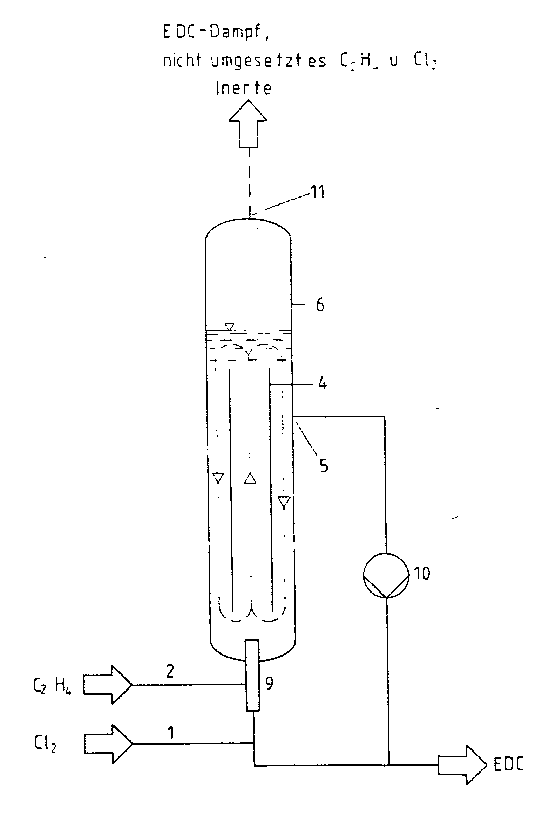

Ein wesentliches gemeinsames Merkmal der für diesen Reaktionstyp bekannten Vorrichtungen ist darin zu sehen, daß die im Produktstrom mehr oder weniger teilweise gelösten Reaktanden Chlor und Ethylen grundsätzlich von unten in den Reaktor eingeleitet werden, mit der in Zirkulation gebrachten flüssigen Reaktionsphase in einem Innenrohr zentral nach oben transportiert werden, dabei an die Phasengrenze zwischen flüssigem Produkt und Dampfraum am Kopf des Reaktors gelangen und teilweise aus der katalysatorhaltigen, flüssigen Reaktionsphase entweichen oder auch zum Teil mit der abwärts führenden Flüssigkeitsbewegung außerhalb des Innenrohres wieder nach unten transportiert werden. Bei dieser Verfahrensweise kann das unerwünschte und den Reaktionsumsatz beeinflussende Heraustreten der gasförmigen Reaktanden aus der flüssigen Reaktionsphase in den Dampfraum des Reaktors nicht vollständig vermieden werden. In Fig. 1 ist schematisch eine bekannte Vorrichtung dargestellt. Sie besteht in der gezeigten Ausführung aus einem zylindrischen Reaktionsgefäß 6, einer Zuleitung der Reaktanden, wie z.B. einer Zuleitung 1 mit Chlor und einer Zuleitung 2 mit gasförmigem Ethylen, welche in einem Verteiler 9 gemischt und über diesen von unten in den Reaktor eingeleitet werden, einem Innenrohr 4, einem Austritt 5 für das Reaktionsprodukt und die im Reaktionsprodukt gelösten bzw. mit diesem dispergierten Bestandteile, einer Pumpe 10 für den Transport der aus dem Reaktor austretenden flüssigen Phase und einem Austritt 11 für die gasförmigen Bestandteile.

Unter konstanten Reaktionsbedingungen, wie z.B. Konzentrationen (Gasanteil), Temperatur usw., wird dieser Entzug der gasförmigen Reaktanden aus der flüssigen Reaktionsphase maßgebend von der Blasengröße und -verteilung im Innenrohr, der Aufwärtsgeschwindigkeit der Gas-Flüssig-Dispersion im Innenrohr und der Flüssigkeitsstandhöhe bestimmt. Die zur Erzeugung einer hohen volumenspezifischen Stoffaustauschfläche erforderliche Energiedissipationsdichte kann wie in der EP—OS 0075 742 und der DE—PS 33 40 624 (Stand der Technik im Sinne von Art. 54.(3) EPU) beschrieben erreicht werden (zum Begriff der Energiedissipationsdichte siehe Ullmanns Enzyklopädie der technischen Chemie, 4 Aufl., Bd. 3 1973, S. 358—360). In diesem Zusammenhang ist insbesondere bei Gas/Flüssig-Dispersionen, die zur Koaleszenzneigen, wie beispielsweise die Ethylen/EDC-Dispersion, der Aspekt einer homogenen Verteilung bei hinreichend größer Energiedissipationsdichte in der flüssigen Phase von Bedeutung. Die Forderung wird von den Verfahren gemäß den genannten Schutzrechten nur zum Teil erfüllt, so daß bei stöchiometrischer Zugabe nicht umgesetzte gasförmige Reaktanden im Gasraum am Kopf des Reaktors anfallen.

Der Erfindung liegt die Aufgabe zugrunde, ein Verfahren und eine Vorrichtung zu entwickeln, bei der die vorher beschriebenen Nachteile einer inhomogenen Energiedissipationsdichte während der Reaktion und das unerwünschte Austreten der Reaktanden aus der Reaktionsphase nicht auftreten und damit eine höhere Raumzeitausbeute in der Vorrichtung, ermöglicht wird.

Diese Aufgabe wird gemäß den kennzeichnenden Merkmalen des Patentanspruchs 1 gelöst. In den Unteransprüchen sind vorteilhafte Ausgestaltungen der Erfindung dargestellt.

Gemäß der Erfindung wird die flüssige mit der gasförmigen Phase in einem statischen Mischer dispergiert und anschließend in einen Reaktor eingeleitet. Mit dem erfindungsgemäßen Verfahren wird erericht, daß die Dispergierung und Induzierung der Reaktion mit einer Energiedissipationsdichte von mehr als 10 kW/m$^3$ bis zu einem.

Umsatz von mindestens 60% erfolgt. Die erhaltene Gas/Flüssig-Dispersion wird derart von oben in ein Innenrohr des vollständig mit flüssigem Reaktionsprodukt gefüllten Reaktors eingeleitet, daß durch Impulsaustausch der Reaktorinhalt eine Zirkulationsbewegung zentral nach unten und in den Randzonen über eine Umlenkung nach oben ausführt. Die nahezu vollständig umgesetzten Reaktanden verlassen den Reaktor an der tiefsten Stelle als nahezu blasenfreie Reaktionsflüssigkeit. Bei der erfindungsgemäßen Vorrichtung werden die geometrischen Verhältnisse zwischen dem Innenrohr- und dem Reaktordurchmeser so gewählt, daß der Flüssigkeitsstrom im Innenrohr mit einer Geschwindigkeit nach unten strömt, die gleich der oder größer ist als die Steiggeschwindigkeit der entsprechenden Gasblasen mittlerer Größe beim Verlassen des statischen Mischers, um eine Blasenkoaleszenz und damit eine Phasentrennung im Reaktor zu vermeiden. Für die Strömung außerhalb des Innenrohres gilt ebenfalls die Bedingung, daß die Geschwindigkeit des—in diesem Fall nach oben gerichteten—Flüssigkeitsstroms größer als die Steiggeschwindigkeit der in diesem Strom enthaltenen Gasblasen mittlerer Größe ist.

Dies wird besonders einfach durch die vorzugsweise Ausgestaltung der Erfindung gemäß den Ansprüchen 2, 5 und 6 erreicht. Die Gas/Flüssig-Dispersion durchströmt nach dem Verlassen des Mischers eine konische Düse. Das Verhältnis des Durchmessers Di des Innenrohres zum Durchmesser Dü des Düsenaustritts dieser Düse liegt im Bereich von $10<(Di/Dü)^2<80$. Das Verhältnis der Länge Li des Innenrohres zum Durchmesser Di des Innenrohres liegt im Bereich $10 \leqslant Li/Di \leqslant 30$, das Verhältnis des Durchmessers Di des Innenrohres zum Durchmesser D des Reaktors im Bereich $0,4<Di/D<0,9$.

Statt der konischen Düse kann auch ein anderer Hohlkörper mit verengtem Querschnitt bei der erfindungsgemäßen Vorrichtung verwendet werden.

Vorzugsweise beträgt beim erfindungsgemäßen Verfahren gemäß Anspruch 3, außerhalb des statischen Mischers im Betriebszustand im Reaktor die Energiedissipationsdichte mindestens 0,05 kW/m³.

Das erfindungsgemäße Verfahren wird nach Anspruch 4, vorzugsweise mit Chlor als in der Reaktionsphase enthaltenem Reaktanden und Ethylen als gasförmigem Reaktanden eingesetzt. Es kann jedoch vorteilhaft auch für andere Reaktionen verwendet werden, wie z.B. Oxidationsreaktionen in der Flüssigphase (beispielsweise Oxidation von i-Butan zu tert. Butylhydroperoxid, p-Xylol zu Terephthalsäure, Ethylen zu Acetaldehyd), Chlorierungen (beispielsweise von Toluol), Carbonylierungen und Hydrierungen.

In vorteilhafter Ausgestaltung nach Anspruch 7 ist der Reaktor zylindrisch. Er kann jedoch auch eine andere Form aufweisen, z.B. konisch ausgebildet sein.

Die Erfindung wird nachfolgend anhand der Zeichnung und eines Beispiels näher erläutert.

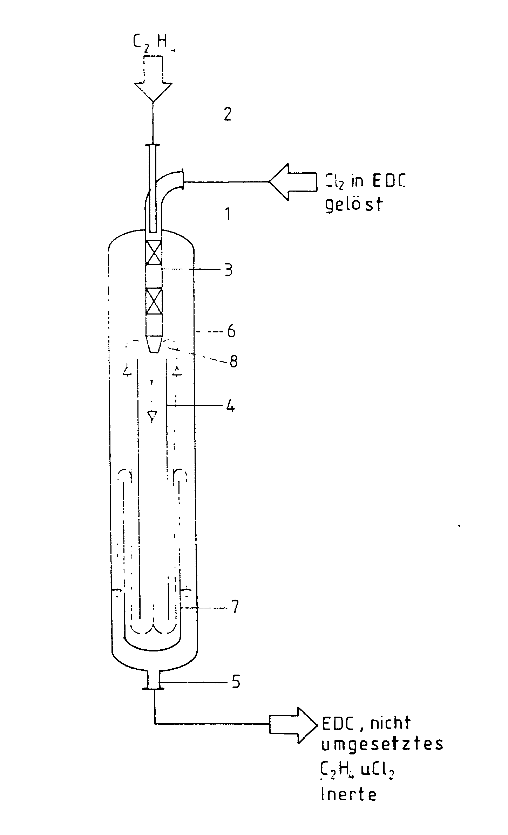

In Fig. 2 ist schematisch die erfindungsgemäß Vorrichtung dargestellt. Sie besteht, in der gezeigten Ausführung aus einem zylindrischen Reaktionsgefäß 6, in welchem das Reaktions- gleich dem Füllvolumen ist, einer von oben nach unten gerichteten Zuleitung der Reaktanden, wie z.B. einer Zuleitung 1 mit in EDC gelöstem Chlor und einer Zuleitung 2 mit gasförmigem Ethylen, einem statischen Mischer 3, in dem die flüssige mit der gasförmigen Phase dispergiert, die Reaktion induziert wird und die Reaktion zum größten Teil unter Aufwendung einer hohen und einheitlichen Energiedissipationsdichte >10 kW/m³ abläuft. Die noch verbleibende reaktive Gas/Flüssig-Dispersion wird vorteilhafterweise über eine konische Düse 8 in ein Innenrohr 4 geleitet, das zentral angeordnet und vollständig mit Reaktionsflüssigkeit, wie z.B. EDC, gefüllt ist. Durch Impulsaustausch zwischen der Dispersion aus dem Mischer 3 und dem Inhalt des Reaktors wird eine Zikulationsströmung erzeugt, die im Innenrohr 4 nach unten gerichtet ist und durch eine Umlenkung 7 außerhalb des Innenrohrs nach oben geführt wird.

Das Verhältnis des Durchmessers Di des Innenrohres zum Durchmesser D des Reaktors wird so gewählt, daß die Strömungsgeschwindigkeit im Innenrohr zumindest gleich der, vorzugsweise größer ist als die Steiggeschwindigkeit der Gasblasen beim Eintritt in das Innenrohr 4. Die mittlere Verweilzeit ergibt sich zwangsläufig für alle Gasblasen aus der Zeitdauer im Innenrohr und im Ringraum außerhalb des Innenrohres und wird von der Länge des Innenrohres und den entsprechenden Zirkulationsgeschwindigkeiten innerhalb und außerhalb des Innenrohres durch die Wahl geeigneter Durchmesser-Verhältnisse $0,4<Di/D<0,9$ bestimmt.

Das Reaktionsprodukt und die mit dem Chlor eingebrachte Kreislaufmenge sowie nicht umgesetzt Anteile an Reaktanden und Inertbestandteile der Reaktanden, wie z.B. Stickstoff, Wasserstoff und Kohlendioxid, werden am Austritt 5 aus dem Reaktor geleitet.

Beispiel:

Eine chlorhaltige EDC-Lösung, bestehend aus 20 m³/h EDC, in der 400 kg/h Chlor gelöst sind, wird über die Zuleitung 1 mit 153 kg/h Ethylen über die Zuleitung 2 in den statischen Mischer 3 geleitet. Durch den Reaktionsumsatz der exothermen Reaktion wird die chlorhaltige EDC-Lösung von 99°C auf 122°C beim Verlassen dieser Reaktionszone erwärmt. Am Austritt 5 des Reaktors wird eine Produkttemperatur von 132°C bei einem Ethylenumsatz von etwa 98% gemessen.

**Patentansprüche**

1. Verfahren zur Durchführung von heterogenen, stofftransportlimitierenden Reaktionen mit einem in der flüssigen, katalysatorhaltigen Reaktionsphase enthaltenen, gegebenenfalls gelösten, und einem gasförmigen Reaktanden mittels einer Vorrichtung, welche einen Reaktor mit einem Innenrohr, einer Zuleitung der Reaktanden und einem Austritt aufweist, dadurch gekennzeichnet, daß

a) beide Reaktanden von oben nach unten zunächst in einen statischen Mischer (3), in welchem eine Dispergierung der flüssigen mit der gasförmigen Phase stattfindet, und anschließend in den Reaktor (6) eingeleitet werden,

b) die Dispergierung und Induzierung der Reaktion mit einer Energiedissipationsdichte von mehr als 10 kW/m³ bis zu einem Umsatz von mindestens 60% im Mischer (3) erfolgt,

(c) die noch nicht vollständig umgesetzte Gas/Flüssig-Dispersion in das Innenrohr (4) des Reaktors (6) geleitet wird und hierbei eine Zirkulationsströmung von oben nach unten bewirkt, deren Geschwindigkeit gleich der Steiggeschwindigkeit der Gasblasen beim Eintritt in das Innenrohr oder größer als diese ist,

(d) die Reaktorflüssigkeit durch eine Umlenkung (7) außerhalb des Innenrohres nach oben geleitet wird, und

(e) der Austritt der Reaktionsprodukte und der Kreislaufprodukte am Boden des Reaktors erfolgt.

2. Verfahren nach Anspruch 1, dadurch gekennzeichnet, daß die Gas/Flüssig-Dispersion nach dem Verlassen des Mischers (3) eine konische Düse (8) durchströmt.

3. Verfahren nach Anspruch 1 oder 2, dadurch gekennzeichnet, daß außerhalb des statischen Mischers (3) im Betriebszustand im Reaktor (6) die Energiedissipationsdichte mindestens 0,05 kW/m³ beträgt.

4. Verfahren nach einem der Ansprüche 1 bis 3, dadurch gekennzeichnet, daß als in der flüssigen Reaktionsphase enthaltener Reaktand Chlor und als gasförmiger Reaktand Ethylen eingesetzt wird.

5. Vorrichtung zur Durchführung des Verfahrens nach einem der Ansprüche 1 bis 4, welche einen Reaktor mit einem Innenrohr, eine Umlenkung (7) außerhalb des Innenrohres, eine Zuleitung der Reaktanden und einem Austritt aufweist, dadurch gekennzeichnet, daß sich im oberen Teil des Reaktors (6) ein statischer Mischer befindet, an dessen unterem Ende eine konische Düse angebracht ist, wobei das Verhältnis des Durchmessers Die des Innenrohrs (4) zum Durchmesser Dü des Düsenaustritts der Düse (8) im Bereich von $10 < (Di/Dü)^2 < 80$ liegt.

6. Vorrichtung nach Anspruch 5, dadurch gekennzeichnet, daß das Verhältnis der Länge Li des Innenrohres (4) zum Durchmesser Di des Innenrohres (4) im Bereich von $10 \leqslant Li/Di \leqslant 30$ und das Verhältnis des Durchmessers Di des Innenrohres (4) zum Durchmesser D des Reaktors (6) im Bereich von $0,4 < Di/D < 0,9$ liegt.

7. Vorrichtung nach Anspruch 5 oder 6, dadurch gekennzeichnet, daß der Reaktor (6) zylindrisch ist.

**Revendications**

1. Procédé de mise en oeuvre de réactions hétérogènes à transport de matières limité, avec un réactif contenu, éventuellement dissous, dans la phase liquide contenant du catalyseur, et avec un réactif gazeux, au moyen d'un dispositif présentant un réacteur avec un tube intérieur, une amenée de réactifs et une sortie, caractérisé par le fait que

a) les deux réactifs sont introduits de haut en bas, d'abord dans un mélangeur statique (3) dans lequel s'effectue une dispersion des phases liquide et gazeuse, puis dans le réacteur (6);

b) la dispersion et l'induction de la réaction s'effectuent dans le mélangeur (3), avec une densité de dissipation d'énergie de plus de 10 kW/m³, jusqu'à une transformation d'au moins 60%;

c) la dispersion gaz-liquide encore incomplètement transformée est amenée dans le tube intérieur (4) du réacteur (6), un courant de circulation étant ainsi produit du haut vers le bas avec une vitesse égale ou supérieure à la vitesse d'ascension des bulles de gaz à l'entrée dans le tube intérieur;

d) par un moyen de déviation (7), le liquide du réacteur est dirigé vers le haut à l'extérieur du tube intérieur, et;

e) la sortie des produits de réaction et des produits de circulation s'effectue au fond du réacteur.

2. Procédé selon la revendication 1, caractérisé par le fait qu'après avoir quitté le mélangeur (3) la dispersion gaz-liquide passe par un ajutage conique (8).

3. Procédé selon la revendication 1 ou 2, caractérisé par le fait qu'en régime de fonctionnement, la densité de dissipation d'énergie dans le réacteur (6), à l'extérieur du mélangeur statique (3), est d'au moins 0,05 kW/m³.

4. Procédé selon l'une des revendications 1 à 3, caractérisé par le fait que l'on utilise le chlore en tant que réactif contenu dans la phase liquide de réaction, et l'éthylène en tant que réactif gazeux.

5. Dispositif de mise en oeuvre du procédé selon l'une des revendications 1 à 4, présentant un réacteur avec un tube intérieur, des moyens de déviation (7) à l'extérieur du tube intérieur, une conduite d'amenée de réactifs et une sortie, caractérisé par la présence, dans la partie supérieure du réacteur (6), d'un mélangeur statique à l'extrémité inférieure duquel est agencé au ajutage conique, le rapport du diamètre Di du tube intérieur (4) au diamètre Dü de la sortie de l'ajutage (8) étant dans une plage telle que $10 < (Di/Dü)^2 < 80$.

6. Dispositif selon la revendication 5, caractérisé par le fait que le rapport de la longueur Li du tube intérieur (4) au diamètre Di du tube intérieur (4) est dans une plage telle que $10 \leqslant Li/Di \leqslant 30$, et le rapport du diamètre Di du tube intérieur (4) au diamètre D du réacteur (6) est dans une plage telle que $0,4 < Di/D < 0,9$.

7. Dispositif selon la revendication 5 ou 6, caractérisé par le fait que le réacteur (6) est cylindrique.

**Claims**

1. Process for the carrying out of heterogeneous reactions in which there is limited transporting of materials with a reactant contained, optionally dissolved, in the liquid catalyst-containing reaction phase and a gaseous reactant, by means of an arrangement with has a reactor with an internal tube, a feed pipe for the reactants and an outlet, characterised in that

a) both reactants are initially introduced from top to bottom firstly into a static mixer (3) in which a dispersing of the liquid with the gaseous takes place and then into the reactor (6)

b) a dispersing and inducing of the reaction takes place with an energy dissipation density of more than 10 kW/m³ to obtain a conversion of at least 60% in mixer (3),

c) the not yet completely reacted gas/liquid dispersion is conducted into the internal tube (4) of the reactor (6) and here there operates a circulation flow from top to bottom whose speed is equal to the rate of climb of the gas bubbles at the entry into the internal tube or is greater than this,

d) the reactor liquid is led upwardly outside the internal tube via a deflector (7) and

e) the discharge of the reaction products and the circulation products takes place at the base of the reactor.

2. Process according to Claim 1, characterised in that the gas/liquid dispersion flows through a conical nozzle (8) after leaving the mixer (3).

3. Process according to Claim 1 or 2, characterised in that the energy dissipation density amounts to at least 0.5 kW/m$^3$ external to the static mixer (3) when the reactor (6) is in operation.

4. Process according to one of Claims 1 to 3, characterised in that chlorine is employed as reactant present in the liquid reaction phase and ethylene is employed as gaseous reactant.

5. Arrangement for carrying out the process according to one of Claims 1 to 4, which has a reactor with an internal tube, a deflector (7) outside to the internal tube, a feed pipe for the reactants and an outlet, characterised in that, in the upper part of the reactor (6), there is be found a static mixer at whose lower end is attached a conical nozzle, with the ratio of the diameter Di of the inner tube (4) to the diameter Du of the nozzle outlet of the nozzle (8) lying in the region of $10 < (Di/Du)^2 < 80$.

6. Arrangement according to Claim 5, characterised in that the ratio of the length Li of the internal tube (4) to the diameter Di of the internal tube (4) lies in the region of $10 \leqslant Li/Di \leqslant 30$ and the ratio of the diameter Di of the internal tube (4) to the diameter D of the reactor (6) lies in the region of $0.4 < Di/D < 0.9$.

7. Arrangement according to Claim 5 or 6, characterised in that the reactor (6) is cylindrical.

**EP 0 185 868 B1**

EDC-Dampf,
nicht umgesetztes $C_2H_4$ u $Cl_2$
Inerte

Fig 1

1

Fig. 2